# EUROPEAN PATENT APPLICATION

(11) **EP 0 610 638 A1**
(43) Date of publication of application: **17.08.1994**
(21) Application number: 93403111.3
(22) Date of filing: 21.12.1993
(51) Int. Cl.: A61F 5/44, A61F 5/451, A61F 5/455

(54) **An incontinence aid assembly**

(30) Priority: 13.01.1993 IL 104376; 08.12.1993 IL 107940
(71) Applicant: A.M.C. ADVANCED MEDICAL CONCEPTS Ltd., Yavne 70600 (IL)
(72) Inventor: Goldenberg, Lior, Rehovot 76288 (IL)
(74) Representative: Portal, Gérard

(57) **Abstract**

An ecologically friendly, non-invasive incontinence aid assembly to be worn by a person suffering from incontinence. The assembly comprises a urine collector unit (11), a urine storage container (23), a flushing liquid container (19), pumps for the urine (12) and the flushing fluid (20) and an air pump (16) for sweeping the urine collector unit with air. In operation urine is temporarily collected in the urine collector from which it is pumped into the urine storage container. The urine collector unit is washed with the flushing liquid and dried by air sweeping. The assembly is suitable for use over an extended period of time without any need for servicing and is not accompanied by unpleasant odors and may be anatomatically fitted for use by either males or females.

## Description

### FIELD AND BACKGROUND OF THE INVENTION

The present invention concerns an incontinence aid assembly.

Incontinence is a problem affecting a significant part of the world population, generally estimated to be from 10 to 13%, the affected population including elderly persons, pregnant women, persons suffering from degenerative diseases and others. At the present time, the problem is generally handled by the use of catheters or diapers. Catheters which are introduced through the urethra into the bladder are very uncomfortable and may cause irritation and/or infection. Diapers are uncomfortable, expensive, require frequent changing and may cause dermatitis, decubitus ulcers, etc. In addition, the non-expendable nature of most diapers adds to the ever increasing ecological problem resulting from non-expendable waste.

It is the object of the present invention to provide a novel, non-invasive and ecologically friendly assembly for use by incontinent persons which can be worn for an extended period of time without the need for any servicing.

### SUMMARY OF THE INVENTION

According to the present invention there is provided an incontinence aid assembly to be worn by a person suffering from incontinence underneath the clothing, comprising a collector unit for intercepting and temporarily collecting the wearer's urine, urine storage means for the accumulation of intercepted urine, and pumping means for withdrawing urine from said collector means into said liquid storage means.

The urine storage means are preferably so designed that in use there always remains an air space above the body of urine therein.

Optionally the assembly according to the invention further includes a flushing liquid container holding a body of flushing liquid, and means for intermittently flushing the said collector unit with flushing liquid in order to remove urine residues and maintain the desired hygienic conditions of said collector. The flushing liquid may be water or a physiological solution and may also include a suitably dosed amount of a drug, e.g. an antiseptic.

If desired, the assembly according to the invention may comprise means for withdrawing urine vapors from said urine collector unit and injecting them into said body of flushing liquid. Where the said urine storage means are so designed that in use an air space always remains above the body of urine therein, the assembly may further include means for withdrawing vapor from said air space and injecting it into said body of flushing liquid. In this way the development of an unpleasant urine smell is suppressed.

The withdrawal of vapors from the collector unit and the air space of the urine storage means may occur separately or simultaneously and be intermittent or simultaneous.

Furthermore, the device may, if desired, also include means for sweeping with air and drying the collector and the adjacent urogenital region of the wearer.

The collector unit in an assembly according to the invention may be so designed as to fit indiscriminately male and female users. Alternatively the collector means may be gender specific and distinct collector units for male and female users may be provided. Typically, a collector unit designed for use by a female subject may be in form of a bowl and one for use by a male subject may comprise a condom-like component.

The collector unit should be made of a non-absorbent bio-compatible material and preferably be pliable so as to make it adjustable to the shape of the body part around the urogenital region. Examples of suitable materials are rubber and various plastic materials.

The pumping means for the withdrawal of urine from the collector means to the liquid storage means and also the pumping means for flushing and for air sweeping, are preferably actuated automatically either in a pre-programmed fashion or in response to sensor means associated with the collector unit.

The said urine storage means may, if desired, comprise manually operable means for the evacuation of accumulated urine and be designed for repeated use. Alternatively, the storage means may be disposable but even so, manually operable urine evacuation means may be provided for interim urine evacuation.

It is thus seen that the invention affords a significant departure from the prior art in that it provides a non-invasive device for the accumulation in a hygienic fashion of involuntarily discharged urine over an extended period of time, say about twelve hours and more, with no need for intermediary servicing.

The amount of discharged urine can be readily assessed from the amount of liquid accumulating in said liquid storage means, making due allowance for the addition of flushing liquid, and the invention thus provides a cheap and reliable method for observation and calculation of the fluid balance of a person suffering from incontinence.

The invention also provides for use in an inconsistence aid assembly, a collector unit as herein described.

### DESCRIPTION OF THE DRAWINGS

For better understanding, the invention will now be described, by way of example only, with reference to the next drawings in which:
**Fig. 1** is an isometric view of underpants fitted with an incontinence aid assembly according to the present invention;
**Fig. 2** is a flow diagram illustrating the operation of the assembly of Fig. 1 with a collector unit adapted for use by a female subject;
**Fig. 3** is a plan view of the collector unit in the assembly of Fig. 2, drawn to a larger scale;
**Fig. 4** is a plan view of a collector assembly adapted for use by a male subject; and
**Fig. 5** is a section along line V-V of Fig. 4.

### DESCRIPTION OF SPECIFIC EMBODIMENTS

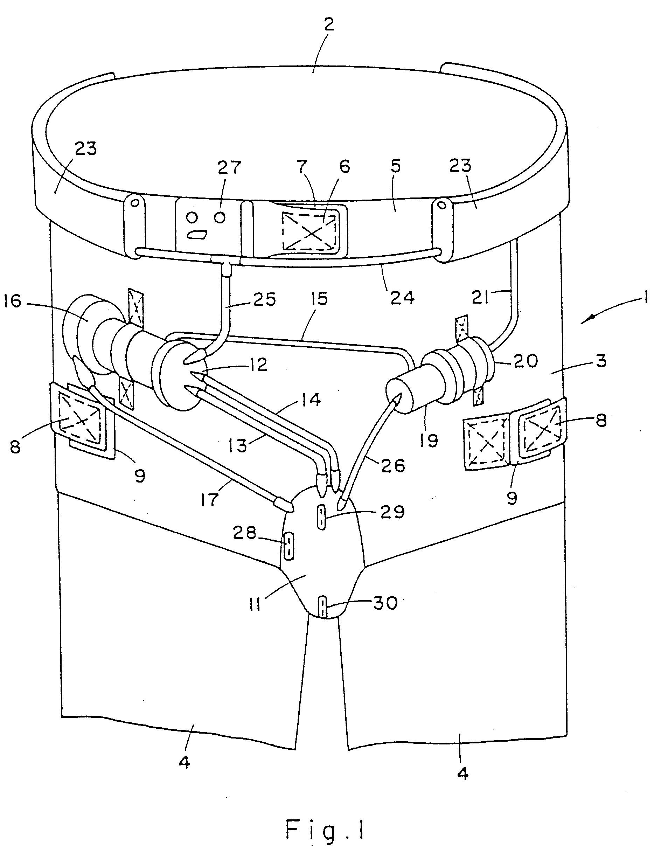

Fig. 1 shows diagrammatically an incontinence aid assembly according to the invention. As shown, the assembly is mounted on underpants 1 having a waist portion 2, a main portion 3 and two pants 4. The waist portion includes a belt 5 having a buckle 6 cooperating with a suitable counterpart such as an anchor patch 7. At the main part 3 there are provided two identical tightening straps of which only the buckles 8 are visible and which are similar as buckle 7 of belt 5 and cooperate each with an anchor patch 9.

The main portion 3 of underpants 1 carries a collector unit 11 located between the two pants 4 and adapted to adequately cover the urogenital region of the wearer; a first pump 12 with associated urine withdrawal pipe 13, flushing liquid delivery pipe 14 and a flushing liquid supply pipe 15; a second, air pump 16 with an associated air delivery pipe 17; and a flushing liquid container 19 with an associated third pump 20 and a vapor intake tube 21. As shown, the components 12, 16, 19 and 20 are fastened to the main part 3 of underpants 1 by means of suitable straps.

Pump 12 is shiftable between two opposite modes of operation, a first one in which urine is pumped out of the collector unit 11 and a second one in which flushing liquid is injected into the collector unit.

The belt 5 carries two identical urine containers 23 linked to each other by a pipe 24 from which a urine delivery pipe 25 branches off. Preferably, the design of containers 23 is such that there always remains a vapor space above the urine therein and that vapor space is linked to the flushing liquid container 19 via the vapor intake tube 21 and pump 20.

Belt 5 further carries an electrical unit generally designated 27, holding actuator means for actuating the various pumps and any other electrical parts included in the assembly. Electrical unit 27 includes a dry battery for electric power supply and a control processor device for the automatic actuation of the pumps in response to signals produced by sensors 28, 29 and 30 associated with the urine collector unit 11. For the sake of clarity of illustration, the various electric wires which connect pumps 12, 16 and 20 as well as sensors 28, 29 and 30 with the electrical unit 27, are not shown. These connections are in accordance with basic principles of electric wiring and can readily be accomplished by a person skilled in the art on the basis of the teachings of the present invention.

It should further be noted that while in Fig. 1 the various components of the assembly according to the invention are visible, in actual practice the underpants 1 may be padded with the various miniaturized pumps, the pipes, the electrical unit and the electric wiring all embedded within the padding so as to be well cushioned and not be visible from the outside.

The unit storage containers 23 may be disposable or alternatively be designed for repeated use. In either case they will preferably have manually operable evacuation means for interim urine evacuation.

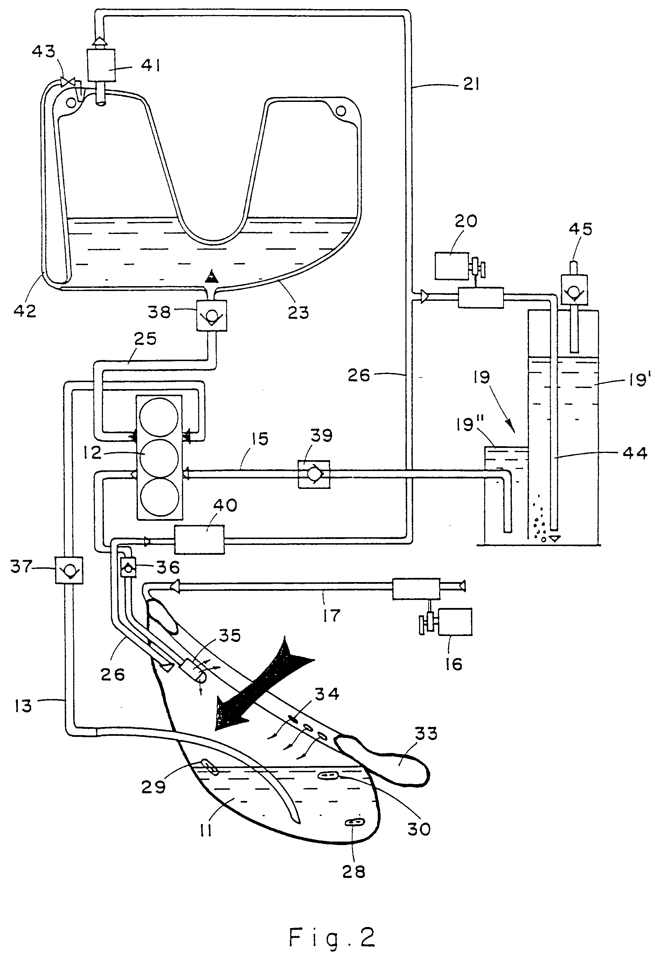

For the description of the operation of an assembly of the kind shown in Fig. 1 attention is now directed to Fig. 2., in which components corresponding to those shown in Fig. 1 are designated with the same numerals.

As shown, the collector unit 11 is in the form of a small bowl having a stiffened upper edge and being circumscribed by an associated inflatable annular tube 33 serving for the isolation of the collector unit and the urogenital region from the surrounding garments and body parts. Tube 33 has a plurality of air ports 34 and comprises air intake means connected to the air delivery pipe 17. In an operation, air is injected intermittently into the annular tube 33 of pump 16 via pipe 17 and is discharged therefrom via parts 34 to sweep and dry the collector unit 11 and the urogenital region.

Additional parts shown in Fig. 2 and not shown in Fig. 1 comprise a liquid spraying nozzle 35, unidirectional valves 36, 37, 38 and 39, filters 40 and 41, a urine evacuation tube 42 with a manually operable control valve 43 and a check valve controlled vent 45. As also shown in Fig. 2 the flushing liquid reservoir 19 is divided into two compartments, a larger one 19' holding the bulk of the flushing liquid and a smaller one 19'' separated from the larger one by a perforated partition and holding an amount of flushing liquid sufficient for one flushing operation.

In operation, urine is intercepted by the collector unit 11 which it enters from above through the opening circumscribed by the inflated annular tube 33, and collects inside the bowl as shown. The collected urine is detected by one or more of the sensors 28 to 30 whereby pump 12 is activated in its first mode in which urine is withdrawn from the collector vessel 11 via duct 13 and check valves 37, 38 and is delivered into the storage vessel 23 via the urine delivery pipe 25. Evacuation of the collector vessel 11 is sensed by one of the sensors 28 to 30 whereupon pump 16 is activated and air is injected into the inflatable tube 33 and from there via ports 34 into the collector unit. In this way both the collector unit 11 and the urogenital region are swept with air and are sufficiently dried.

A major problem conventionally encountered in connection with incontinence is the development of an unpleasant smell. A substantial factor in the controlling of said unpleasant smell is the neutralization of urine vapor which develops in the urine collector unit such as unit 11 in Fig. 1, and in any air space of the urine storage means such as in urine storage containers 23 in Fig. 1. In accordance with the present invention, the suppression of said unpleasant smell may be achieved by withdrawing the urine vapor from collector unit 11 and urine storage containers 23 and injecting them into the body of flushing liquid in reservoir 19. Accordingly, when the urine inside the collector vessel has reached a certain level which is sensed by sensors 28 to 30, pump 20 is activated to suck vapors from container 23 via duct 21, filter 41 and duct 44, and from collector unit 11 via duct 26 and filter 40. The combined vapor streams are injected into the main compartment 19' of the reservoir 19 and are dissolved in the flushing liquid therein. The flushing liquid in reservoir 19 is capable of absorbing the amount of urine vapor collected during one operational period of the assembly (i.e. the period of time between one replacement of the flushing liquid in reservoir 19 with fresh flushing liquid to the next replacement therein) while still remaining efficient as effective flushing liquid. Any excessive pressure building up inside compartment 19' is released via the check valve controlled vent 45. Although as here illustrated, chamber 19'' of **reservoir** 19 is linked only to one of the containers 23, it is possible, if desired, to link it to both containers in parallel or in series.

As an alternative to the sensor controlled intermittent withdrawal of vapor from urine collector unit 11 and the air space of container(s) 23, the operations may be continuous.

Simultaneously with the activation of pump 20, pump 12 is switched from the urine withdrawal mode to the liquid injection mode in which flushing liquid is withdrawn from the small compartment 19'' of reservoir 19 via duct 15 and check valve 39 and is injected into the air space of collector unit 11 via the spraying nozzle 35. The operation of pump 20 and the injection mode of pump 12 are interrupted after a predetermined period of time whereupon pump 12 reverts into the urine withdrawal mode and upon activation by the sensors, continues to withdraw urine and any accumulated flushing liquid from the collector unit 11 into container 23, as specified.

It is to be noted that flushing of said collector vessel 11 may also be achieved by a flushing dedicated pump rather than pump 12. By yet another alternative, flushing of the collector vessel 11 may be achieved with no pumping at all. In such a case, an additional pressure sensitive check valve (designated herein as *"flushing valve"*) is installed in the bottom region of reservoir 19. When the pressure building up inside reservoir 19 as a result of vapor injection reaches a certain predetermined activation level, (which obviously must be lower than the pressure at which vent 45 yields), the flushing valve yields and an amount of flushing liquid is discharged into duct 15 and is sprayed into the urine collector unit 11, until the pressure inside reservoir 19 drops to a level at which the flushing valve snaps back into the shut state.

The container or containers 23 may be emptied periodically by unhooking duct 42 and manually opening the associated control valve 43.

The capacity of the urine container vessel or vessels 23 and the amount of flushing liquid in reservoir 19 may be designed for a continuous 12-hour or even 24-hour operation. At the end of the design period, containers 23 are emptied or replaced and fresh flushing liquid is filled into the reservoir 19. The collector unit 11 which is of a material not affected by the urine, e.g. rubber or plastic material, need not be replaced but may be periodically removed for cleansing.

If desired, in the assembly of Fig. 2 there may be more than one urine withdrawal pipe 13 associated with the collector unit 11 so as to make allowance for any shifting of the urine inside unit 11 in consequence of the subject changing position from standing to lying or vice versa.

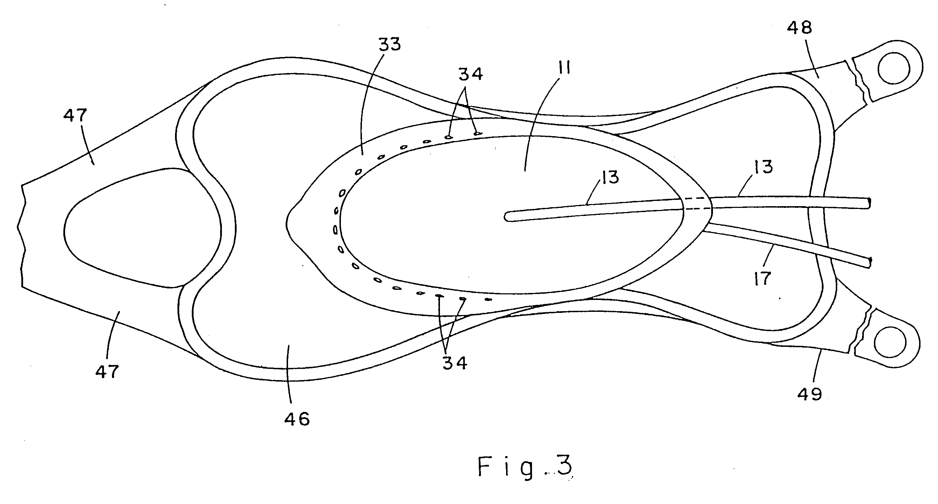

Attention is now directed to Fig. 3 which is a plan view of the urine collector unit 11 shown in Fig. 2 and which is adapted for use by a female subject. As shown, unit 11 has a urine receiving vessel which is circumscribed by the inflatable tube 33 which has one or more air discharge ports 34 and is associated with the urine withdrawal pipe 13 and the air injection pipe 17. As further shown in Fig. 3 (but not in Fig. 2) the collector unit 11 is mounted on a supporting sheet 46 fitted with straps 47, 48 and 49 by which it can either be tied directly to the body of the wearer or else be connected to underpants of the kind shown in Fig. 1 which carry all the remaining parts of the assembly.

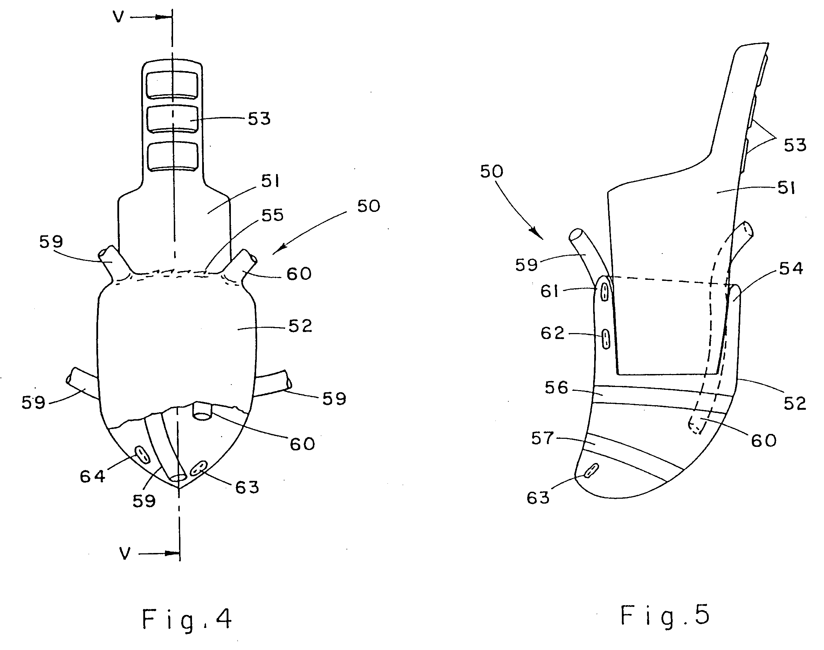

Attention is now directed to Figs. 4 and 5 which show a urine collector unit for incorporation in an assembly according to the invention, for use by a male subject. The unit, generally designated 50, comprises two main components 51 and 52 both made of bio-compatible material. Component 51 is preferably an air permeable fabric which is applied in a diaper-like fashion around the wearer's penis, adjusted to the individual size and attached to the wearer's undergarment, e.g. of the kind shown in Fig. 1, by means of anchor patches 53. Once component 51 is snugly fitted it provides protection to the urogenital region from possible pressure and friction which may result from the wearing and operation of the remaining parts of the device.

Component 52 is a condom like bag made of elastic material, e.g. rubber. In operation, the open end part of bag 52 is fitted around the first component 50, if desired with a fold 54 as shown in Fig. 5 whereby the length of component 52 may be adjusted according to need. As bag 52 is made of elastic material its size is adjustable. For further adjustment there may be provided some folds 55 as shown in Fig. 4 which provide for lateral expansion so as to avoid that the permanent pressure on the penis be too high.

In order to ensure that component 52 remains secured throughout, some circumferential tightening straps such as straps 56 and 57 may be provided.

Urine is evacuated via pipes 59 and flushing liquid is injected via pipe 60.

There are further provided sensors 61, 62, 63 and 64 having functions similar to sensors 28 to 30 in Fig. 2.

## Claims

1. An incontinence aid assembly to be worn by a person suffering from incontinence underneath the clothing, comprising a collector unit (11) for intercepting and temporarily collecting the wearer's urine, urine storage means (23) for the accumulation of intercepted urine, and pumping means (12) for withdrawing urine from said collector means into said liquid storage means.

2. An assembly according to Claim 1, further comprising a flushing liquid container (19) and means (12, 14) for intermittently flushing said collector unit with liquid from said flushing liquid container.

3. An assembly according to Claim 1, wherein said urine storage means (23) are so designed that in use there always remains an air space above the body of urine therein.

4. An assembly according to Claim 1, comprising means (20, 26) for withdrawing urine vapors from said urine collector unit (11) and injecting them into said body of flushing liquid.

5. An assembly according to Claims 2 and 3, further including means (20, 21) for withdrawing vapors from said air space and injecting them into said body of flushing liquid.

6. An assembly according to any one of Claims 1 to 5, including an air pump (16) and ducting means (17) associated herewith for the delivery of air to said collector unit (11) whereby the unit and the urogenital region are swept and residual liquid is removed.

7. An assembly according to any one of Claims 1 to 6, comprising an electric unit (27) with pump actuation and control processor means, signal generating sensor means (28, 29, 30) associated with said urine collector means (11) being linked to said processor means, whereby the pumps (12, 16, 20) are activated in response to conditions inside the urine collector means (11).

8. An assembly according to any one of Claims 2 to 7, wherein said pumping means for withdrawing urine from said collector means (12) are capable of operating in two modes, a first mode in which urine is withdrawn from the collector means and a second mode in which flushing liquid is injected into the collector means (11).

9. An assembly according to any one of Claims 2 to 7, comprising a dedicated flushing pump.

10. An assembly according to any one of Claims 4 to 7, wherein said flushing liquid storage container (19) has a pressure sensitive flushing valve capable of yielding to a predetermined pressure building up inside the container by said vapors, whereby an amount of flushing liquid is discharged from the container.

11. An assembly according to any of Claims 1 to 10, wherein said urine collector means (11) are gender specific.

12. An assembly according to Claim 11, comprising a female specific collector unit having a bowl-shaped receptacle in association with an isolating inflatable tube (33).

13. An assembly according to Claim 12, wherein said inflatable tube (33) comprises at least one air discharge port (34) for the delivery of flushing air into said bowl-shaped receptacle.

14. An assembly according to Claim 11, comprising a male specific collector unit having a condom-like component (52).

15. A female specific collector unit for use in an incontinence aid assembly, comprising a bowl-shaped receptacle in association with an isolating inflatable tube (33), which receptacle holds signal generating sensors (28, 29, 30) and comprises liquid inlets and outlets.

16. A female specific collector unit according to Claim 15, wherein said inflatable annular tube has at least one port (34) for the delivery of flushing air into said bowl-shaped receptacle.

17. A male specific collector unit for use in an incontinence aid assembly, comprising a condom-like component (52) holding signal generating sensors (63, 64) and fitted with inlets and outlets.
